# EUROPÄISCHE PATENTSCHRIFT

(11) **EP 1 656 405 B1**
(45) Veröffentlichungstag und Bekanntmachung des Hinweises auf die Patenterteilung: **12.03.2008**
(21) Anmeldenummer: 04741270.5
(22) Anmeldetag: 26.07.2004
(51) Int. Cl.: C08G 18/09, C08G 18/16, B01J 31/02, C07D 213/76

(54) **NEUE KATALYSATOREN FÜR DIE SELEKTIVE ISOCYANATDIMERISIERUNG**
NOVEL CATALYSTS FOR SELECTIVE ISOCYANATE DIMERISATION
NOUVEAUX CATALYSEURS POUR LA DIMERISATION SELECTIVE D'ISOCYANATES

(30) Priorität: 07.08.2003 DE 10336184
(43) Veröffentlichungstag der Anmeldung: 17.05.2006
(73) Patentinhaber: Bayer MaterialScience AG, 51368 Leverkusen (DE)
(72) Erfinder: KÖCHER, Jürgen, 40764 Langenfeld (DE); LAAS, Hans-Josef, 51519 Odenthal (DE)
(86) Internationale Anmeldenummer: PCT/EP2004/008343
(87) Internationale Veröffentlichungsnummer: WO 2005/016984

(56) Entgegenhaltungen:
- EP-A- 0 317 744
- WO-A-03/029217
- DE-A- 19 754 749
- FR-A- 2 816 939
- US-A- 3 829 525
- US-A- 5 227 493
- DATABASE CA [Online] CHEMICAL ABSTRACTS SERVICE, COLUMBUS, OHIO, US; 1962, KOSTSOVA, A. G. ET AL: "Properties of .alpha.-aminopyridides of alkanesulfonic acids" XP002305422 gefunden im STN Database accession no. 58:33271 & ZHURNAL OBSHCHEI KHIMII , 32, 1009-10 CODEN: ZOKHA4; ISSN: 0044-460X, 1962,

## Beschreibung

Die vorliegende Erfindung betrifft die Verwendung von Sulfonamid-Salzen als Dimerisierungskatalysatoren für Isocyanate sowie ein Verfahren zur Herstellung von oligomeren Isocyanaten unter Verwendung der erfindungsgemäßen Katalysatoren.

Da monomere Diisocyanate als Vernetzer in Polyurethan-Beschichtungssystemen aufgrund ihrer Flüchtigkeit und ihrer toxikologischen Eigenschaften nicht eingesetzt werden können, bedient man sich in der Regel der höhermolekularen Derivate z.B. auf Uretdion- Isocyanurat-, Biuret-, Urethan- oder Allophanatbasis. Eine Übersicht zu diesen Polyisocyanaten und ihrer Herstellungsweise ist exemplarisch in J. Prakt. Chem/Chem. Ztg. 1994, 336, 185-200 gegeben. Auf dem Gebiet der lichtechten Lacke und Beschichtungsmittel werden üblicherweise Polyisocyanate auf Basis aliphatischer und/oder cycloaliphatischer Diisocyanate verwendet.

Die Oligomerisierung (üblicherweise Dimerisierung, Trimerisierung) von Isocyanaten zu Uretdionen, Isocyanuraten oder Iminooxadiazindionen ist eine lange bekannte und in der Praxis gut eingeführte Methode zur Modifizierung in der Regel difunktioneller, niedermolekularer C₁-C₃₀ Isocyanate. Speziell jedoch für die Isocyanatdimerisierung gibt es bisher nur wenige brauchbare Katalysatoren mit hoher Aktivität und Selektivität, die sich auch für den Einsatz im technischen Maßstab eignen.

Ein Überblick über die technisch relevanten Dimerisierungsverfahren des Standes der Technik und die dabei zum Einsatz gelangenden Katalysatoren bzw. Katalysatorsysteme wird in J. Prak. Chem. 336 (1994) 185-200 gegeben. Nachteilig an den dort angegebenen Katalysatoren ist ihre zum Teil nur unzureichende katalytische Aktivität und mangelnde Selektivität bezogen auf die Dimerbildung, weshalb es insbesondere für den technischen Einsatz den Bedarf an neuen verbesserten Systemen gibt.

EP-A 45 995 beschreibt die Verwendung spezieller peralkylierter Aminophosphine als Katalysatoren zur selektiven Dimerisierung von Isophorondiisocyanat (IPDI) (Trimerisatanteil < 2 Gew.-%). Ein wesentlicher Nachteil an diesen Verbindungen ist jedoch ihre Oxidationsempfindlichkeit zu Phosphorsäureamiden (z.B. Hexamethylphosphorsäuretriamid (HMPT)), die ein hohes krebserzeugendes Potential besitzen, was für einen breiten technischen Einsatz prohibitiv ist.

Die EP-A 317 744 beschreibt ein Verfahren zur Herstellung linearer (cyclo)aliphatischer Uretdione durch Katalyse mit 4-Dimethylaminopyridinen, wie z. B. 4-Dimethylaminopyridin (4-DMAP). Auch dieses Verfahren liefert lineare, nahezu Isocyanuratgruppen-freie IPDI-Uretdione.

Sowohl die Katalysatoren nach EP-A 45 995 als auch nach EP-A 317 744 weisen nur eine mäßige katalytische Aktivität auf und sind gegenüber Isocyanaten inaktiv, deren NCO-Gruppen ausschließlich an sekundären C-Atomen gebunden sind (z.B. 4,4'-Düsocyanatodicyclohexylmethan).

Eine verbesserte katalytische Aktivität zeigen die in WO 02/92658 beschriebenen Azolatanionen, wobei besonders cycloaliphatische Diisocyanate mit einer hohen Selektivität zu dimeren Uretdionen umgesetzt werden.

FR-A 2 816 939 offenbart die selektive Dimerisierung von Polyisocyanaten wie EPDI durch Katalysatoren, die Sulfonylimid-Strukturen enthalten, wobei Umsätze von etwa 20 % erzielt werden.

Aufgabe der vorliegenden Erfindung war es daher, neue Katalysatoren für die Dimerisierung zur Verfugung zu stellen, die neben einer hohen Selektivität auch eine deutlich verbesserte katalytische Aktivität zeigen und im technischen Maßstab einsetzbar sind.

Diese Aufgabe wird gelöst durch die Verwendung von Sulfonamid-Salzen der Formel.(I) 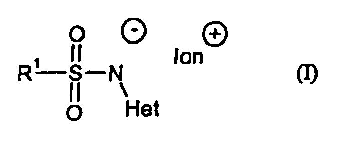 als Katalysatoren für die Dimerisierung von Isocyanaten, in der
- R¹: ein aliphatischer, cycloaliphatischer, aromatischer oder araliphatischer gegebenenfalls Heteroatom-haltiger Rest ist, der gegebenenfalls substituiert ist,
- Het: ein Rest ausgewählt aus der Gruppe Thiazolyl-, Benzthiazolyl-, 2-Pyrimidyl-, 4-Pyrimidyl-, 2-Pyridyl- und 4-Pyridyl- ist und der gegebenenfalls substituiert ist und
- Ion⁽⁺⁾: ein anorganisches oder organisches Kation ist.

Gegenstand der Erfindung ist daher die Verwendung von Sulfonamid-Anionen der allgemeinen Formel (I) zur Dimerisierung von Isocyanaten.

Bevorzugt werden als Katalysatoren Verbindungen der allgemeinen Formel (II) eingesetzt 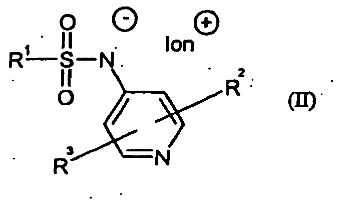 in der
- R¹: ein gesättigter oder ungesättigter aliphatischer oder cycloaliphatischer Rest mit bis zu 24 Kohlenstoffatomen und gegebenenfalls bis zu 3 Heteroatomen aus der Gruppe Sauerstoff, Schwefel oder Stickstoff ist, der gegebenenfalls weiter substituiert ist,
- R², R³: unabhängig voneinander gleiche oder verschiedene Gruppen ausgewählt aus der Gruppe Wasserstoff, Halogen, Cyanid, Nitro, und Dialkylamino sowie gegebenenfalls substituierte Alkyl-, Aryl-, Alkoxy-, Aryloxyreste sind und
- Ion⁽⁺⁾: ein Alkalikation wie beispielsweise Li⁺, Na⁺ und K⁺, ein Erdalkalikation wie beispielsweise Mg²⁺ und Ca²⁺ oder ein Ammonium- bzw. Phosphoniumion der allgemeinen Formel (III) ist, 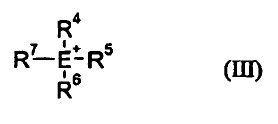 in welcher
E für Stickstoff oder Phosphor steht,
R⁴, R⁵, R⁶ unabhängig voneinander Wasserstoff oder gleiche oder verschiedene gesättigte oder ungesättigte aliphatische oder cycloaliphatische und gegebenenfalls Substituententragende Reste mit bis zu 24 Kohlenstoffatome und gegebenenfalls bis zu 3 Heteroatomen aus der Gruppe Sauerstoff, Schwefel und Stickstoff sind, wobei diese Reste gegebenenfalls durch Halogenatome oder Hydroxygruppen substituiert sind und
R⁷ der Definition der Reste R⁴, R⁵, R⁶ entspricht oder für einen Rest der Formel (IV) steht 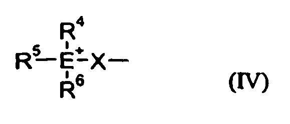 in welchem
X ein zweibindiger, gegebenenfalls substituierter aliphatischer, cycloaliphatischer, araliphatischer oder aromatischer C₁ - C₁₂ Rest ist und
R⁴, R⁵, R⁶ und E die oben genannte Bedeutungen haben.

Besonders bevorzugt werden als erfindungsgemäße Katalysatoren Verbindungen der Formel V eingesetzt 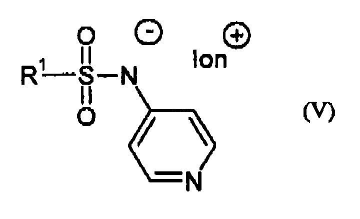 in der
- R¹: ein aliphatischer oder cycloaliphatischer C₁-C₁₈ Rest ist, der gegebenenfalls bis zu drei Heteroatome aus der Gruppe Sauerstoff, Schwefel, Stickstoff enthält und gegebenenfalls Substituenten aus der Gruppe Halogen, Nitro, Cyanid, Dialkylamino, Alkyl-, Aryl-, Alkoxy-, Aryloxy- aufweist,
- Ion⁽⁺⁾: ein Alkalikation oder ein einwertiges Ammonium- oder Phosphoniumkation der allgemeinen Formel (III) ist, in welcher
E für Stickstoff oder Phosphor steht und
R⁴, R⁵, R⁶, R⁷ unabhängig voneinander ein gesättigter aliphatischer oder cycloaliphatischer oder gegebenenfalls substituierter aromatischer oder araliphatischer Rest mit bis zu 18 Kohlenstoffatomen sind.

Ein weiterer Gegenstand der Erfindung ist ein Verfahren zur Herstellung von oligomeren Isocyanaten, bei dem
a) ein oder mehrere organische Verbindungen einer mittleren NCO-Funktionatität ≥ 1 in Anwesenheit
b) eines Katalysators enthaltend ein oder mehrere Sulfonamid-Salze der Formel (I) und
c) optional Lösungsmitteln
oligomerisiert werden.

In das erfindungsgemäße Verfahren können in Komponente a) alle dem Fachmann bekannten aliphatischen, cycloaliphatischen, araliphatischen und/oder aromatischen Isocyanate einer mittleren NCO-Funktionalität ≥ 1, bevorzugt ≥ 2 einzeln oder in beliebigen Mischungen untereinander eingesetzt werden, wobei es unerheblich ist, ob diese nach Phosgen- oder phosgen-freien Verfahren hergestellt wurden.

Bevorzugt werden aliphatische, cycloaliphatische und/oder araliphatische Isocyanate der vorstehend genannten Art eingesetzt, wobei diese ein Kohlenstoffgrundgerüst (ohne die enthaltenen NCO-Gruppen) von 3 bis 30, bevorzugt 4 bis 20 Kohlenstoffatomen aufweisen.

Besonders bevorzugte Verbindungen der Komponente a) entsprechen der vorstehend genannten Art mit aliphatisch und/oder cycloaliphatisch gebundene NCO-Gruppen wie beispielsweise Bis-(isocyanatoalkyl)ether, Bis- und Tris-(isocyanatoalkyl)-benzole, -toluole, sowie -xylole, Propandiisocyanate, Butandiisocyanate, Pentandiisocyanate, Hexandiisocyanate (z.B. Hexamethylendiisocyanat, HDI). Heptandiisocyanate, Octandiisocyanate, Nonandiisocyanate (z.B. Trimethyl-HDI (TMDI) in der Regel als Gemisch der 2,4,4- und 2,2,4-Isomeren), Nonantri-isocyanate (z.B. 4-Isocyanatomethyl-1,8-octandiisocyanat), Dekandiisocyanate, Dekantriisocyanate, Undekandiisocyanate, Undekantriisocyanate, Dodecandiisocyanate, Dodecantriisocyanate, 1,3- sowie 1,4-Bis-(isocyanatomethyl)cyclohexane (H₆XDI), 3-Isocyanatomethyl-3,5,5-trimethylcyclohexylisocyanat (Isophorondiisocyanat, IPDI). Bis-(4-isocyanatocyclohexyl)methan (H₁₂MDI), Bis-(isocyanatomethyl)norboman (NBDI) oder 3(4)-Isocyanatomethyl-1-methyl-cyclohexylisocyanat (IMCI).

Ganz besonders bevorzugte Verbindungen der Komponente a) sind Hexamethylendiisocyanat (HDI), Trimethyl-HDI (TMDI), 2-Methylpentan-1,5-diisocyanat (MPDI), Isophorondiisocyanat (IPDI), 1,3- sowie 1,4-Bis(isocyanatomethyl)cyclohexan (H₆XDI), Bis(isocyanatomethyl)norbornan (NBDI), 3(4)-Isocyanatomethyl-1-methyl-cyclohexylisocyanat (IMCI) und/oder 2,4'- bzw. 4,4'-Bis(isocyanatocyclohexyl)methan (H₁₂MDI) oder Gemische dieser Isocyanate.

Die anteilige Verwendung monofunktioneller Isocyanate ist gegebenenfalls in besonderen Fällen ebenfalls möglich.

Im erfindungsgemäßen Verfahren beträgt die Menge des Katalysators b) 0,01 bis 10 mol%, bevorzugt 0,05 bis 5 mol%, besonders bevorzugt 0,1 bis 3 mol% bezogen auf die Menge der Komponente a), wobei sich hier die mol%-Angaben auf die gesamte Stoffmenge in mol des eingesetzten Isocyanates der Komponente a) bezieht.

Bevorzugt werden als Katalysator b) des erfindungsgemäßen Verfahrens ausschließlich Sulfonamid-Salze der Formel (I) eingesetzt.

Der Katalysator b) kann ungelöst als reine Verbindung oder in Form einer Lösung im erfindungsgemäßen Verfahren eingesetzt werden. Das Lösungsmittel ist dabei so zu wählen, dass es den Katalysator zwar molekular oder ionisch dissoziiert löst, nicht jedoch das bzw. die Sulfonamidanion(en) durch chemische Reaktionen in seiner/ihrer Zusammensetzung und/oder molekularen Struktur dabei verändert wird/werden. Gleichzeitig muss das Lösungsmittel gegenüber NCO-Funktionen entweder inert sein oder darf mit Isocyanaten nur unter Ausbildung von Harnstoff-, Biuret-, Urethan- oder Allophanat-Gruppen reagieren.

Sofern der Katalysator b) als Lösung eingesetzt wird, werden bevorzugt geradkettige oder verzweigte Alkohole mit einer mittleren OH-Funktionalität ≥ 1 und 1 bis 20, bevorzugt 1 bis 10 C-Atomen wie beispielsweise Methanol, Ethanol, 1- sowie 2-Propanol, die isomeren Butanole, 2-Ethylhexanol, 2-Ethylhexan-1,3-diol, 1,3- sowie 1,4-Butandiol oder 1-Methoxy-2-propanol verwendet.

In einer bevorzugten Ausführungsform der Erfindung wird der Katalysator b) als Lösung eingesetzt.

Im erfindungsgemäßen Verfahren können als Komponente c) gegebenenfalls Lösungsmittel mitverwendet werden, wobei bevorzugt außer dem gegebenenfalls verwendeten Katalysatorlösungsmittel keine weiteren Lösungsmittel als Komponente c) eingesetzt werden.

Das erfindungsgemäße Verfahren wird bevorzugt bei Temperaturen von 0 bis 100°C, besonders bevorzugt 20 bis 100°C durchgeführt.

Selbstverständlich kann falls notwendig das Verfahren auch unter erhöhtem oder vermindertem Druck durchgeführt werden.

Das erfindungsgemäße Verfahren kann sowohl kontinuierlich als auch diskontinuierlich geführt werden. Unter einem kontinuierlichen Verfahren wird z.B. die Herstellung in einem Rohrreaktor oder mit Hilfe von Kesselkaskaden verstanden, während diskontiuierliche Verfahren z.B. Verfahren in einem Kessel (Batch-Verfahren) oder einem Kolben sind.

In einer bevorzugten Ausführungsform der Erfindung wird die NCO-Oligomerisierung bis zu einem Umsatz von 10-60 mol-% bezogen auf die Gesamtmenge der ursprünglich vorhandenen NCO-Gruppen geführt, die Oligomerisierungsreaktion abgebrochen und nicht umgesetztes Isocyanat z.B. durch Destillation gegebenenfalls unter vermindertem Druck abgetrennt, wobei das oligomerisierte Isocyanat als Harz erhalten wird.

Zum Abbruch der Oligomerisierungsreaktion eignen sich prinzipiell alle dem Fachmann bekannten Techniken (J. Prakt. Chem./Chem. Ztg. 1994, 336, 185-190). Dazu gehört die Entfernung des Katalysators z.B. durch Extraktion oder Filtration gegebenenfalls unter Zuhilfenahme eines adsorptiv wirkenden Trägermaterials, die Inaktivierung des Katalysatorsystems durch thermische Behandlung und/oder durch Zugabe von Säuren oder Säurederivaten wie Benzoylchlorid, Phthaloylchlorid, phosphinige-, phosphonige- oder phosphorige Säure, Phosphin-, Phosphon- oder Phosphorsäure oder die sauren Ester der oben genannten Phosphor-enthaltenden Säuren. Bevorzugte Abstopper sind Mono- oder Dialkylphosphate wie (Di)butylphosphat, (Di)octylphosphat oder (Di)trihexylphosphat, Schwefelsäure oder ihre sauren Ester oder Sulfonsäuren, wie bevorzugt Methansulfonsäure und p-Toluolsulfonsäure, sowie Alkylester von Sulfonsäuren, wie bevorzugt p-Toluolsulfonsäuremethylester.

Die zur Abstoppung der Reaktion benötigte Menge des Katalysatorgiftes richtet sich dabei nach Menge des aktiven Katalysators. Im Allgemeinen werden 50-150 mol-% Abstopper bezogen auf die ursprünglich eingesetzte Katalysatormenge verwendet, bevorzugt ist der Einsatz äquimolarer Mengen Abstopper bezogen auf die eingesetzte Katalysatormenge.

Die nach dem erfindungsgemäßen Verfahren erhaltenen Polyisocyanate lassen sich durch die üblichen Verfahren des Standes der Technik, wie z.B. Dünnschichtdestillation, Extraktion, Kristallisation und/oder Molekulardestillation isolieren und reinigen. Sie fallen dabei als farblose oder nur schwach gefärbte Flüssigkeiten oder Feststoffe an.

Besonders vorteilhaft an den erfindungsgemäßen Katalysatoren zur Isocyanat-Oligomerisierung ist ihre hohe Selektivität zur Bildung von Uretdionstrukturen, wobei sie hochaktiv sind. Besonders im Fall der cycloaliphatischen Isocyanate zeigen die erfindungsgemäßen Katalysatoren zusätzlich eine für ionische Katalysatoren hohe Neigung zur Bildung von NCO-Dimeren.

Die erfindungsgemäß hergestellten Polyisocyanate stellen vielseitig verwendbare Ausgangsmaterialien zur Herstellung von Polymeren dar, wie z.B. gegebenenfalls geschäumten Kunststoffen oder Polyurethan-Lacken, insbesondere zur Herstellung von Ein- und ZweikomponentenPolyurethanlacken, Beschichtungsmitteln, Klebstoffen und Zuschlagstoffen für die Anwendung auf Materialien wie z.B. Holz, Kunststoff, Leder, Metall, Papier, Beton , Mauerwerk, Keramik und Textil.

### Beispiele

Die Prozentangaben des Umsatzes berechnen sich durch Division der Menge an umgesetztem Isocyanat durch die Gesamtmenge des eingesetzten Isocyanats multipliziert mit 100. Alle weiteren Prozentangaben sind, soweit nicht anders vermerkt, als Gewichtsprozente zu verstehen.

### Verwendete Abkürzungen :

- DMSO:: Dimethylsulfoxid
- i-PrOH:: Isopropanol
- n-Bu:: n-Butyl-
- Hex:: n-Hexyl-

Die Messung von Farbzahlen erfolgte nach der DIN-Norm 53995 (Gerät: LICO 200, Dr. Lange GmbH, Berlin, DE).

Die Ermittlung des NCO-Gehaltes der in den Beispielen und Vergleichsbeispielen beschriebenen Harze erfolgte durch Titration gemäß DIN 53 185.

Die dynamischen Viskositäten der Polyisocyanatharze wurden bei 23°C mit dem Viskosimeter VT 550, Platte-Kegel Messanordnung PK 100, der Fa. Haake (Karlsruhe, Deutschland) bestimmt. Durch Messungen bei unterschiedlichen Schergeschwindigkeiten wurde sichergestellt, dass das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Die hergestellten Rohprodukte wurden mittels ¹H-NMR-Spektroskopie auf einem DPX 400 der Firma Bruker, Karlsruhe, Deutschland, bei einer ¹H-Resonanzfrequenz von 400 MHz untersucht. Als Referenz für die ppm-Skala wurde Tetramethylsilan als interner Standard verwendet.

Zur Bestimmung des Isocyanat-Umsatzes wurden 20 bis 40 mg der hergestellten Reaktionsmischungen in 3 ml Chloroform gelöst und mit Hilfe der Gelpermeationschromatographie (Säule MZ-Gel Sdplus 500A 5 µm, MZ-Analysentechnik, Mainz, Deutschland) untersucht. Auf eine Katalysatordeaktivierung konnte aufgrund der hohen Verdünnung der Messlösung verzichtet werden. Aus der ermittelten Menge an monomerem Isocyanat kann der NCO-Umsatz bzw. die Harzausbeute berechnet werden.

Die anschließende Bestimmung der Selektivität des eingesetzten Katalysators wurde durch Untersuchung der möglichen Strukturtypen 1 bis 3 durchgeführt.

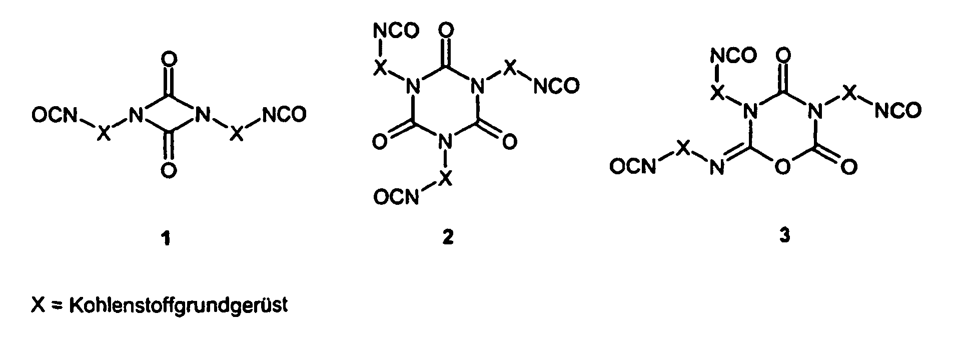

Dazu wurden 30 µl des Reaktionsgemischs zwischen KBr-Platten IR-spektroskopisch (Spektrometer: Arid-Zone^{®} der Firma Bomem, Québec, Kanada, Scanzahl 10, Auflösung 2 cm⁻¹) vermessen. Anhand der Schwingungen bei 1760 cm⁻¹ (Strukturtyp 1), 1690 cm⁻¹ (Strukturtyp 2) und 1780 cm⁻¹ (Strukturtyp 3) kann eine Bildung der Strukturtypen 1 - 3 nachgewiesen werden. Für den Fall, dass mehr als nur ein Strukturtyp gebildet wurde, wurden zur quantitativen Auswertung ¹³C-NMR-Messungen durchgeführt und die Produktmengen über die Signalintegration berechnet.

Für die ¹³C-NMR-Analytik wurden 0,5 ml des jeweiligen Reaktionsgemischs mit, bezogen auf die eingesetzten Katalysatormengen, stöchiometrischen Mengen an Di-n-butylphosphat versetzt, um den Katalysator zu deaktivieren und eine Weiterreaktion zu unterbinden. Durch Zugabe von deuteriertem Chloroform wurden etwa eine Konzentration von 50 Gew.-% Harz eingestellt. Die Messungen erfolgten auf einem DPX 400 der Fa. Bruker, Karlsruhe, DE bei einer ¹³C-Resonanzfrequenz von 100 MHz. Als Referenz für die ppm-Skala wurde Tetramethylsilan als interner Standard verwendet. Daten für die chemische Verschiebung der infrage kommenden Verbindungen sind der Literatur entnommen (vgl. Die Angewandte Makromolekulare Chemie 1986, 141, 173-183 und darin zit. Lit) bzw. durch Vermessung von Modellsubstanzen gewonnen worden.

Katalysatorherstellung

### Beispiel 1: Herstellung, von n-Butyl-N-4-pyridyl-sulfonamid

In 540 ml THF wurden 51,0 g 4-Aminopyridin (0,542 mol) und 75,0 ml Triethylamin (54,8 g, 0,542 mol) bei 50°C gelöst. Zu dieser Lösung wurden ebenfalls bei 50°C 70,3 ml n-Butansulfonsäurechlorid (84,9 g, 0,542 mol) innerhalb von 1 h zugetropft. Nach 19 h Rühren bei 50°C wurde das Reaktionsgemisch mit 500 ml Methylenchlorid verdünnt und zweimal mit 500 ml 1 N NaOH extrahiert. Die wässrige Phase wurde mit konzentrierter HCl auf pH 6-7 eingestellt und dann mit 500 ml Methylenchlorid extrahiert. Die organische Phase wurde mit Magnesiumsulfat getrocknet, und das Methylenchlorid abdestilliert. Die erhaltenen 30 g Rohprodukt wurden aus Acetonitril umkristallisiert. Man erhielt 19,0 g sauberes Produkt. Die Konstitution der Verbindung wurde mittels NMR-Spektroskopie gesichert.

### Beispiel 2: Herstellung von Methyl-N-4-pyridyl-sulfonamid

In 72 ml Dimethylformamid wurden 9,9 g 4-Aminopyridin (104,8 mmol) und 14,5 ml Triethylamin (10,6 g, 104,8 mmol) bei Raumtemperatur gelöst. Zu dieser Lösung wurden ebenfalls bei Raumtemperatur 8,1 ml Methansulfonsäurechlorid (12,0 g, 104,8 mmol) innerhalb von einer Stunde zugetropft. Aufgrund der exothermen Reaktion musste die Temperatur durch eine Eisbadkühlung eingehalten werden. Nach 20 h Rühren bei Raumtemperatur wurde das Lösungsmittel im Vakuum abdestilliert. Der verbleibende Rückstand wurde in 100 ml Methylenchlorid und in 150 ml 1 N NaOH aufgenommen. Die organische Phase wurde abgetrennt. Die wässrige Phase wurde mit konzentrierter HCl vorsichtig auf pH 4-5 gestellt, dann mit 100 ml EtOH und 100 ml Methylenchlorid versetzt. Nach Abtrennen der organischen Phase wurde die wässrige Phase noch dreimal mit 100 ml EtOH und 100 ml Methylenchlorid versetzt. Die gesammelten organischen Phasen wurden vom Lösungsmittel befreit, und das verbleibende Rohprodukt aus Acetonitril umkristallisiert. Man erhielt 3,2 g Produkt. Die Konstitution der Verbindung wurde mittels NMR-Spektroskopie gesichert.

### Beispiel 3: Herstellung von n-Propyl-N-4-pyridyl-sulfonamid

In 500 ml THF wurden 47,6 g 4-Aminopyridin (0,506 mol) und 70,0 ml Triethylamin (51,1 g, 0,506 mol) bei 50°C gelöst. Zu dieser Lösung wurden ebenfalls bei 50°C 57,3 ml n-Propansulfonsäurechlorid (72,2 g, 0,506 mol) innerhalb von 1 h zugetropft. Nach 19 h Rühren bei 50°C wurde das Reaktionsgemisch mit 500 ml Methylenchlorid verdünnt und einmal mit 500 ml 1 N NaOH extrahiert. Die wässrige Phase wurde mit konzentrierter HCl auf pH 5-6 eingestellt, mit 100 ml Ethanol versetzt und dann fünfmal mit 200 ml Methylenchlorid extrahiert. Diese organischen Phasen wurden mit Magnesiumsulfat getrocknet, und dann das Methylenchlorid abdestilliert. Die erhaltenen 74 g Rohprodukt wurden aus Acetonitril umkristallisiert. Man erhielt 20,8 g sauberes Produkt. Die Konstitution der Verbindung wurde mittels NMR-Spektroskopie gesichert.

### Beispiel 4: Herstellung der Sulfonamid-Salze

Eine Lösung von 4,7 mmol des betreffenden Sulfonamids in 8 ml Methanol wurde bei Raumtemperatur zu 0,9 ml einer 30 %-igen Na-Methylatlösung in Methanol (4,7 mmol) zugetropft und für eine Stunde bei Raumtemperatur nachgerührt. Anschließend wurden 4,7 mmol des Ammonium- bzw. Phosphoniumsalzes zugegeben und ebenfalls für eine Stunde bei Raumtemperatur nachgerührt. Dann wurde das ausgefallene NaCl abfiltriert, das Filtrat im Vakuum vom Lösungsmittel befreit und der so erhaltene Rückstand im Vakuum getrocknet.

Für das Beispiel 4i wurde ein zehnfacher Ansatz durchgeführt.

| Edukte | 4a | 4b | 4c | 4d | 4e | 4f | 4g | 4h | 4i |
|---|---|---|---|---|---|---|---|---|---|
| Sulfonamid aus Beispiel 1 | 1,0 g | 1,0 g | 1,0 g | 1,0 g | | | | | |
| Sulfonamid aus Beispiel 2 | | | | | 0,8 g | 0,8 g | 0,8 g | 0,8 g | |
| Sulfonamid aus Beispiel 3 | | | | | | | | | 10 g |
| [Bu₄N]Cl, 61,4 % in i-Propanol | 2,1 g | | | | 2,1 g | | | | |
| [Bu₄P]Cl, 71,4 % in i-Propanol | | 2,0 g | | | | 2,0 g | | | |
| [Bu₃P-C₁₄H₂₉]Cl | | | 2,1 g | | | | 2,1 g | | |
| [Hex₃P-C₁₄H₂₉]Cl | | | | 2,4 g | | | | 2,4 g | 27,9 g |

### Beispiele 5 bis 7: Erfindungsemäße Oligomerisierunssreaktionen

### Allgemeine Vorschrift

In Glasgefäßen mit Septumverschluss wurden die in den Tabellen 1-3 angegebenen Mengen an reinem Katalysator eingewogen. Dann wurde jedes Gefäß zweimal evakuiert und mit Argon gefüllt. Mit Hilfe einer Spritze wurden dann die angegebenen Mengen Diisocyanat über das Septum zugegeben.

Für den Fall, dass der Katalysator als Lösung verwendet wurde (Beispiele 5b,c,d,e, 6i, 6j und 7h), wurde das Reaktionsgefäß mit Septumverschluss zweimal evakuiert und mit Argon gefüllt. In das so vorbereitete Gefäß wurde mit Hilfe einer Spritze jeweils 5 ml Diisocyanat eingefüllt und danach unter Rühren die entsprechenden Mengen an Katalysator in dem angegebenen Lösungsmittel zugegeben.

Das erhaltene Reaktionsgemisch wurde anschließend in einem Ölbad oder in einem Rührheizblock (z.B. Variomag Reaktionsblock Typ 48.2/RM der Firma H&P Labortechnik GmbH, Oberschleißheim, Deutschland) unter den in den Tabellen 1 bis 3 angegebenen Bedingungen umgesetzt.

Die Analytik erfolgte wie oben angegeben.

**Tabelle 1: Resultate der erfindungsgemäßen HDI Oligomerisierung**

| Bsp | Kat. | Menge [mol-%] | Einsatzform | Zeit [h] | T [°C] | Umsatz [%] | Typ 1 [mol-%] | Typ 2 [mol%] |
|---|---|---|---|---|---|---|---|---|
| 5a | 4a | 0,05 | 100 %-ig | 0,08 | 40 | 33 3 | 27 | 73 |
| 5b | 4c | 0,08 | 0,1 M / DMSO | 3,5 | 40 | 26 | 49 | 51 |
| 5c | 4c | 0,1 | 0.1M/DMSO | 3,5 | 40 | 38 | 42 | 58 |
| 5d | 4d | 0,25 | 1,8 M / i-PrOH | 2 | 40 | 45 | 75 | 25 |
| 5e | 4d | 0,3 | 1,8 M / i-PrOH | 2 | 40 | 45 | 77 | 23 |
| 5f | 4f | 0,4 | 100 %-ig | 0,25 | 40 | 31 | 88 | 12 |
| 5g | 4f | 0,5 | 100 %-ig | 0,25 | 40 | 48 | 89 | 11 |
| 5h | 4g | 0,4 | 100 %-ig | 0,25 | 40 | 26 | 90 | 10 |
| 5i | 4g | 0,5 | 100 %-ig | 0,25 | 40 | 33 | 91 | 9 |
| 5j | 4h | 0,4 | 100 %-ig | 0,25 | 40 | 38 | 86 | 14 |
| 5k | 4h | 0,5 | 100 %-ig | 0,25 | 40 | 43 | 87 | 13 |

**Tabelle 2: Resultate der erfindungsgemäßen IPDI-Oligomerisierung**

| Bsp | Kat. | Menge [mol-%] | Einsatzform | Zeit [h] | T [°C] | Umsatz[%] | Typ 1 [mol-%] | Typ 2 [mol-%] |
|---|---|---|---|---|---|---|---|---|
| 6a | 4a | 0,1 | 100 %-ig | 0,75 | 40 | 34 | 100 | 0 |
| 6b | 4a | 0,15 | 100 %-ig | 0,75 | 40 | 40 | 100 | 0 |
| 6c | 4b | 0,1 | 100 %-ig | 2 | 40 | 36 | 100 | 0 |
| 6d | 4b | 0,15 | 100 %-ig | 2 | 40 | 46 | 100 | 0 |
| 6e | 4c | 0,25 | 100 %-ig | 1 | 40 | 33 | 100 | 0 |
| 6f | 4c | 0,5 | 100 %-ig | 1 | 40 | 47 | 100 | 0 |
| 6g | 4d | 0,25 | 100 %-ig | 0,5 | 40 | 37 | 100 | 0 |
| 6h | 4d | 0,50 | 100 %-ig | 0,5 | 40 | 42 | 100 | 0 |
| 6i | 4d | 0,3 | 1,8M /i-PrOH | 1,5 | 40 | 45 | 100 | 0 |
| 6j | 4d | 0,4 | 1,8 M / i-PrOH | 1,5 | 40 | 45 | 100 | 0 |
| 6k | 4f | 0,25 | 100 %-ig | 1 | 40 | 30 | 100 | 0 |
| 6l | 4f | 0,5 | 100 %-ig | 1 | 40 | 36 | 100 | 0 |
| 6m | 4g | 0,25 | 100 %-ig | 1 | 40 | 33 | 100 | 0 |
| 6n | 4g | 0,5 | 100 %-ig | 1 | 40 | 42 | 100 | 0 |
| 6p | 4h | 0,25 | 100 %-ig | 1 | 40 | 33 | 100 | 0 |
| 6q | 4h | 0,5 | 100 %-ig | 1 | 40 | 48 | 100 | 0 |

**Tabelle 3: Resultate der erfindungsgemäßen H₁₂ MDI-Oligomerisierung**

| Bsp | Kat. | Menge [mol-%] | Einsatzform | Zeit [h] | T [°C] | Umsatz[%] | Typ 1 [mol-%] | Typ 2 [mol-%] |
|---|---|---|---|---|---|---|---|---|
| 7a | 4a | 0,75 | 100 %-ig | 1 | 40 | 25 | 20 | 80 |
| 7b | 4a | 1 | 100 %-ig | 1 | 40 | 31 | 17 | 83 |
| 7c | 4b | 0,25 | 100 %-ig | 23,5 | 40 | 42 | 100 | 0 |
| 7d | 4b | 0,5 | 100 %-ig | 23,5 | 40 | 44 | 100 | 0 |
| 7e | 4c | 0,5 | 100 %-ig | 4 | 40 | 32 | 100 | 0 |
| 7f | 4c | 1,0 | 100 %-ig | 4 | 40 | 37 | 100 | 0 |
| 7g | 4d | 1,0 | 100 %-ig | 21 | 40 | 40 | 100 | 0 |
| 7h | 4d | 1,5 | 1,8 M/i-PrOH | 42 | 40 | 24 | 100 | 0 |
| 7i | 4f | 1 | 100 %-ig | 23 | 40 | 25 | 100 | 0 |
| 7j | 4g | 0,75 | 100 %-ig | 21 | 40 | 24 | 100 | 0 |
| 7k | 4h | 1 | 100 %-ig | 16 | 40 | 21 | 100 | 0 |

### Vergleichsbeispiele 1 bis 3

Die Umsetzung von HDI, IPDI und H₁₂-MDI wurde mit den literaturbekannten Katalysatoren:
- Benzyltrimethylammoniumhydroxid, vgl. EP-A 0 010 589 (verwendet wird das unter dem Handelsnamen Triton^{®} B als 40 %ige methanolische Lösung von der Fa. Aldrich angebotene Produkt),
- Tri-n-butylphosphin, vgl. DE-A 16 70 720 (Katalysator: Cytop^{®} 340, Fa. Cytec, unverdünnt) sowie
- 4-Dimethylaminopyridin, vgl. DE-A 37 39 549 (Katalysator: 4-DMAP, Fa. Aldrich, unverdünnt)
nach oben beschriebenen allgemeinen Vorschrift durchgeführt. Die relevanten Ergebnisse bei Verwendung dieser, nicht erfindungsgemäßen Katalysatoren, sind in den nachfolgenden Tabellen dargestellt.

**Tabelle 4: Vergleichsbeispiele 1 a) und b): Umsetzungen von HDI**

| Nr. | Nr. Katalysator | Kat.-Konz. [mol-%] | Zeit [h] | Temp. [°C] | Umsatz [%] | Typ 1 [mol-%] | Typ 2 [mol-%] | Typ 3 [mol%] |
|---|---|---|---|---|---|---|---|---|
| 1a | Triton^{®}B | 0,035 | 0,25 | 60 | 42,7 | 2,1 | 94,4 | 3,5 |
| 1b | n-Bu₃P | 1,30 | 1,5 | 60 | 40,6 | 69,7 | 15,7 | 14,6 |

**Tabelle 5: Vergleichsbeispiele 2 a) - f): Umsetzungen von IPDI**

| Nr. | Katalysator | Kat.-Konz. [mol%] | Zeit [h] | Temp. [°C] | Umsatz [%] | Typ 1 [mol-%] | Typ 2 [mol-%] |
|---|---|---|---|---|---|---|---|
| 2a | Triton^{®} B | 0,07 | 2,5 | 60 | 43,1 | 2,1 | 97,9 |
| 2b | 4-DMAP | 1,7 | 24 | 40 | 30 | 98,8 | 1,2 |
| 2c | n-Bu₃P | 2 | 5,5 | 40 | 18,7 | 69,3 | 30,7 |

**Tabelle 6: Vergleichsbeispiele 3 a) - c): Umsetzungen von H₁₂=MDI**

| Nr. | Katalysator | Kat.-Konz. [mo%] | Zeit [h] | Temp. [°C] | Umsatz [%] | Typ 1 [mol-%] | Typ 2 [mol-%] |
|---|---|---|---|---|---|---|---|
| 3a | Triton^{®} B | 0,2 | 21,5 | 40 | 51,7 | 1,2 | 98,8 |
| 3b | 4-DMAP | 2 | 456 | 40 | 14,3 | 97,8 | 2,2 |
| 3c | n-Bu₃P | 2 | 48 | 40 | 3,9 | 86,5 | 13,5 |

Wie man sehen kann, ist das salzartig aufgebaute Tetraalkylammoniumhydroxid sehr aktiv, liefert aber nur geringe Uretdionanteile im Produktgemisch. Die beiden kovalent aufgebauten Katalysatoren liefern zwar hohe Uretdionanteile im Produktgemisch, ihre Aktivität ist aber gering, so dass selbst bei Einsatz hoher Katalysatorkonzentrationen, insbesondere bei den cycloaliphatischen Diisocyanaten IPDI und H₁₂MDI nur ein sehr langsamer Umsatz eintritt.

Im Gegensatz zu den Katalysatoren der Vergleichsversuche sind die erfindungsgemäßen Katalysatoren sehr selektive Dimerisierungskatalysatoren bei einer höheren Aktivität, wie der Vergleich der Ergebnisse zur Umsetzung von IPDI zeigt. Für das H₁₂MDI sind die Katalysatoren 4-Dimethylaminopyridin und Tri-n-butylamin der Vergleichsversuche zwar selektiv für die Dimerisierung, aber bei einer sehr geringen Aktivität. Hier haben die erfindungsgemäßen Katalysatoren deutliche Vorteile in der hohen Aktivität bei gleich hoher oder sogar höherer Selektivität für die Dimerisierung.

### Beisipiel 8: Verfahrensbeispiele

### A) Erfindungsgemäß

1000 g (4,50 mol) Isophorondiisocyanat (IPDI) wurden bei 30°C unter trockenem Stickstoff vorgelegt. Unter Rühren wurden dann 15 g (0,022 mol) des Katalysators des Beispiels 4i mit Hilfe einer Infusions-Laborpumpe (KDS 100, KD Scientific, Boston, USA) kontinuierlich über eine Reaktionszeit von 3 Stunden zugegeben. Die Oligomerisierungsreaktion verlief unter diesen Bedingungen ohne erkennbare Exothermie. Nach beendeter Katalysatorzugabe wurde das Reaktionsgemisch 10 Minuten nachgerührt und anschließend der Katalysator durch Zugabe von 5 g (0,024 mol) Dibutylphosphat deaktiviert. Es wurde eine klare, farblose Reaktionsmischung mit einem NCO-Gehalt von 28,5 % erhalten, entsprechend einem Oligomerisierungsgrad von 24,6 %. Das Reaktionsgemisch wurde anschließend mit Hilfe eines Dünnschichtverdampfers bei einer Temperatur von 160°C und einem Druck von 0,3 mbar von überschüssigem Diisocyanat befreit. Es wurde auf diese Weise ein praktisch farbloses Uretdionpolyisocyanat mit einem Gehalt an freien NCO-Gruppen von 17,5 %, einem Gehalt an monomerem IPDI von 0,4 %, einer Viskosität von mehr als 200.000 mPas (23°C) und einer Farbzahl (APHA), bestimmt an einer 10 %-igen Lösung in Methylenchlorid, von 12, erhalten. Das Produkt enthielt nach ¹³C-NMR- und IR-Spektroskopie ausschließlich Uretdiongruppen. Isocyanuratstrukturen waren nicht nachweisbar.

### B) Vergleich (gemäß EP-A 317 744)

1000 g (4,50 mol) Isophorondiisocyanat (IPDI) wurden bei Raumtemperatur unter trockenem Stickstoff und Rühren mit 20 g (0,164 mol) 4-Dimethylaminopyridin (DMAP) als Katalysator versetzt. Nach 20 h wurde die hellgelbe Reaktionsmischung, die einen NCO-Gehalt von 28,7 %, entsprechend einem Oligomerisierungsgrad von 22,6 % aufwies, ohne vorherige Zugabe eines Katalysatorgiftes mit Hilfe eines Dünnschichtverdampfers bei einer Temperatur von 160°C und einem Druck von 0,3 mbar von flüchtigen Bestandteilen befreit. Es wurde ein hochviskoses hellgelbes Uretdionpolyisocyanat mit einem Gehalt an freien NCO-Gruppen von 17,6 %, einem Gehalt an monomerem IPDI von 0,4 % und einer Farbzahl (APHA), bestimmt an einer 10 %-igen Lösung in Methylenchlorid, von 62 erhalten. Das Produkt war laut ¹³C-NMR- und IR-Spektrum frei von Isocyanuratstrukturen.

Der Vergleich belegt die höhere Aktivität des erfindungsgemäßen Katalysators gegenüber Katalysatoren nach EP-A 317 744. Trotz einer erheblich geringeren Katalysatorkonzentration lassen sich in deutlich kürzerer Zeit reine (lineare) Uretdionpolyisocyanate des IPDI herstellen, die sich darüber hinaus durch eine deutlich verbesserte Farbe auszeichnen.

## Patentansprüche

1. Verwendung von Sulfonamid-Salzen der Formel (1) 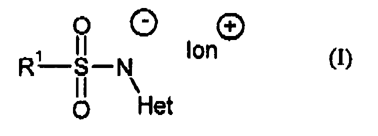 in der
R¹ ein aliphatischer, cycloaliphatischer, aromatischer oder araliphatischer gegebenenfalls Heteroatom-haltiger Rest ist, der gegebenenfalls substituiert ist,
Het ein Rest ausgewählt aus der Gruppe Thiazolyl-, Benzthiazolyl-, 2-Pyrimidyl-, 4-Pyrimidyl-, 2-Pyridyl- und 4-Pyridyl- ist und der gegebenenfalls substituiert ist und
Ion⁽⁺⁾ ein anorganisches oder organisches Kation ist,
zur Dimerisierung von Isocyanaten.

2. Verwendung von Sulfonamid-Salzen gemäß Anspruch 1 zur Dimerisierung von Isocyanaten, **dadurch gekennzeichnet, dass**
R¹ ein aliphatischer oder cycloaliphatischer C₁ - C₁₈ Rest ist, der gegebenenfalls bis zu drei Heteroatome aus der Gruppe Sauerstoff, Schwefel, Stickstoff enthält und gegebenenfalls Substituenten aus der Gruppe Halogen, Nitro, Cyanid, Dialkylamino, Alkyl-, Aryl-, Alkoxy- oder Aryloxy- aufweist,
Het 4-Pyridyl- ist und
Ton⁽⁺⁾ ein Alkalikation oder ein einwertiges Ammonium- oder Phosphoniumkation der allgemeinen Formel (III) ist, 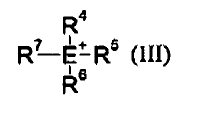 in welcher
E Stickstoff oder Phosphor ist und
R⁴, R⁵, R⁶, R⁷ unabhängig voneinander ein gesättigter aliphatischer oder cycloaliphatischer oder gegebenenfalls substituierter aromatischer oder araliphatischer C₁ - C₁₈ Rest sind.

3. Verfahren zur Dimerisierung von Isocyanaten, bei dem
a) ein oder mehrere organische Verbindungen einer mittleren NCO-Funktionalität ≥ 1 in Anwesenheit
b) eines Katalysators enthaltend ein oder mehrere Sulfonamid-Salze gemäß Anspruch 1 oder 2 und
c) optional Lösungsmitteln
dimerisiert werden.

4. Verfahren zur Dimerisierung von Isocyanaten gemäß Anspruch 3, **dadurch gekennzeichnet, dass** die NCO-Oligomerisierung bei einer Temperatur von 20 - 100°C so lange durchgeführt wird, bis 10-60 mol% aller NCO-Gruppen umgesetzt wurden und die Dimerisierungsreaktion dann durch Zugabe eines Katalysatorgiftes abgebrochen und nicht umgesetztes monomeres Isocyanat durch Destillation abgetrennt wird.

## Claims

1. Use of sulphonamide salts of the formula (I) 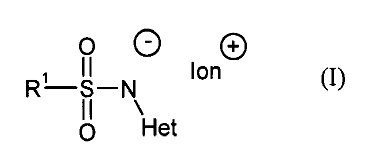 in which
R¹ is an aliphatic, cycloaliphatic, aromatic or araliphatic, optionally heteroatom-containing radical which is optionally substituted,
Het is a radical selected from the group consisting of thiazolyl, benzthiazolyl, 2-pyrimidyl, 4-pyrimidyl, 2-pyridyl and 4-pyridyl and is optionally substituted and
Ion⁽⁺⁾ is an organic or inorganic cation
for dimerizing isocyanates.

2. Use of sulphonamide salts according to Claim 1 for dimerizing isocyanates, **characterized in that**
R¹ is an aliphatic or cycloaliphatic C₁-C₁₈ radical optionally containing up to three heteroatoms from the group consisting of oxygen, sulphur and nitrogen and optionally containing substituents from the group consisting of halogen, nitro, cyanide, dialkylamino, alkyl, aryl, alkoxy and aryloxy,
Het is 4-pyridyl and
Ion⁽⁺⁾ is an alkali metal cation or a monovalent ammonium or phosphonium cation of the general formula (III) 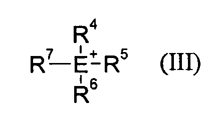 in which
E is nitrogen or phosphorus and
R⁴, R⁵, R⁶ and R⁷ independently of one another are a saturated aliphatic or cycloaliphatic or optionally substituted aromatic or araliphatic C₁-C₁₈ radical.

3. Process for dimerizing isocyanates wherein
a) one or more organic compounds having an average NCO functionality ≥ 1 are dimerized in the presence
b) of a catalyst comprising one or more sulphonamide salts according to Claim 1 or 2 and
c) optionally solvents.

4. Process for dimerizing isocyanates according to Claim 3, **characterized in that** the NCO oligomerization is carried out at a temperature of 20 - 100°C until 10-60 mol% of all the NCO groups have undergone conversion and then the dimerization reaction is terminated by addition of a catalyst poison and unreacted monomeric isocyanate is separated off by distillation.

## Revendications

1. Utilisation de sels de sulfonamides de formule (1) 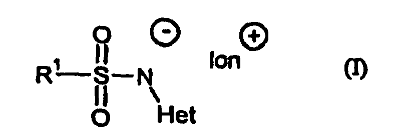 dans laquelle
R¹ est un reste aliphatique, cycloaliphatique, aromatique ou araliphatique contenant éventuellement des hétéroatomes, qui est éventuellement substitué,
Het est un reste choisi dans le groupe constitué par thiazolyle, benzothiazolyle, 2-pyrimidyle, 4-pyrimidyle, 2-pyridyle et 4-pyridyle, et qui est éventuellement substitué, et
Ion⁽⁺⁾ est un cation inorganique ou organique,
pour la dimérisation d'isocyanates.

2. Utilisation de sels de sulfonamides selon la revendication 1 pour la dimérisation d'isocyanates, **caractérisée en ce que**
R¹ est un reste aliphatique ou cycloaliphatique en C₁-C_{18,} qui contient éventuellement jusqu'à 3 hétéroatomes du groupe constitué par l'oxygène, le soufre, l'azote, et qui présente éventuellement des substituants du groupe constitué par halogène, nitro, cyanure, dialkylamino, alkyle, aryle, alcoxy ou aryloxy,
Het est 4-pyridyle, et
Ion⁽⁺⁾ est un cation alcalin ou un cation monovalent d'ammonium ou de phosphonium de formule générale (III) 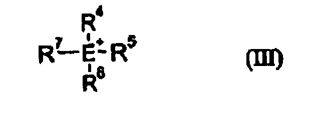 dans laquelle
E est l'azote ou le phosphore et
R⁴, R⁵, R⁶, R⁷ sont indépendamment les uns des autres un reste en C₁-C₁₈
aliphatique ou cycloaliphatique saturé ou aromatique ou araliphatique éventuellement substitué.

3. Procédé de dimérisation d'isocyanates, dans lequel on dimérise
a) un ou plusieurs composés organiques ayant une fonctionnalité NCO moyenne ≥ 1 en présence
b) d'un catalyseur contenant un ou plusieurs sels de sulfonamides selon la revendication 1 ou 2 et
c) de solvants facultatifs.

4. Procédé de dimérisation d'isocyanates selon la revendication 3, **caractérisé en ce que** l'on effectue l'oligomérisation de NCO à une température de 20-100°C jusqu'à ce que 10-60 % en mol de tous les groupes NCO aient réagi, puis on arrête la réaction de dimérisation en ajoutant un poison du catalyseur, et on sépare par distillation l'isocyanate monomère n'ayant pas réagi.
